# EUROPEAN PATENT APPLICATION

(11) **EP 4 379 059 A1**
(43) Date of publication of application: **05.06.2024**
(21) Application number: 22210964.7
(22) Date of filing: 01.12.2022
(51) Int. Cl.: C12P 3/00, C12R 1/01

(54) **NEW THERMOPHILIC BACTERIA STRAINS TO PRODUCE HYDROGEN IN ACIDIC CONDITIONS**

(71) Applicant: Université d'Aix-Marseille, 13007 Marseille (FR); Centre National de la Recherche Scientifique, 75016 Paris (FR); Institut De Recherche Pour Le Développement (IRD), 13002 Marseille 2 (FR)
(72) Inventor: ERAUSO, Gaël, 13009 MARSEILLE (FR); COMBET-BLANC, Yannick, 13009 MARSEILLE (FR); DAVIDSON, Sylvain, 13008 MARSEILLE (FR); PRIME, Anne-Hélène, 13400 AUBAGNE (FR)
(74) Representative: Plasseraud IP

(57) **Abstract**

The present invention relates to new thermophilic anaerobic bacteria strains to produce hydrogen gas in acidic conditions and a method for producing hydrogen by culturing these thermophilic bacteria strains which are Maritinoga sp. BP5C14, DSM 34310, Thermotoga sp. GM6, DSM 34137 and Thermoanaerobacterium sp. BRSS1, DSM 34145.

## Description

### TECHNICAL FIELD

The present invention relates to new thermophilic bacteria strains to produce hydrogen in acidic conditions and a method for producing hydrogen by culturing these thermophilic bacteria strains in presence of substrates.

### BACKGROUND OF THE INVENTION

The cost of conventional energy sources has increased dramatically in the last few years, and the use of many conventional energy sources, such as oil, coal, and nuclear power, has been demonstrated to be harmful to the environment. Many clean alternative energy sources have been developed or proposed.

Hydrogen (H₂) is considered a green and sustainable alternative to traditional fossil fuels and is capable of mitigating greenhouse gas emissions. Using hydrogen in fuel cells or combustion engines produces heat and electricity with water as the main waste. As the electric current, the industrial processes of hydrogen production used until now are energy-consuming and still cause pollution. Therefore, emphasis must be given to the biological production of energy from renewable sources.

Biological synthesis of H₂ can use a wide range of organic substrates as feedstocks, including agro-industrial wastes and algal biomass, and may operate under various environmental conditions. In addition, high temperatures help to improve the solubilization of substrates, reduce fermentation time, and lower contamination risks.

The pH at which the bacterial incubation process is conducted is also extremely important, and greatly affects hydrogen gas yields. The best hydrogen production performances have been reached at neutral pH. Generation of volatile fatty acids as by-products decreases the pH of the fermentation process, which can be controlled by alkaline agent adding. However, the latter causes significant environmental impact (Ochs et al. Journal of cleaner production, 2010, 18-Supplement 1: S88-S94) and restricts water recirculation due to accumulation of salts. Moreover, addition of caustic agents like sodium hydroxide incurs significant costs (Ljunggren et al. Biotechnology for Biofuels. 2011. 4(31)).

### SUMMARY OF THE INVENTION

The inventors screened and characterized new thermophilic bacteria strains having the capacity to produce hydrogen under acidic conditions. These strains are particularly useful to avoid pH control during the hydrogen production process.

The present disclosure relates to a method for producing hydrogen comprising the step of culturing at least one thermophilic bacteria in presence of a substrate at a pH lower than 6 and in temperature conditions allowing the bacteria to grow and produce hydrogen; wherein said thermophilic bacteria is selected from the group consisting of: *Marinitoga sp.* BP5C14 strain deposited with DSMZ on 10 February 2022; Accession No. DSM 34310, *Thermotoga sp.* GM6-5-3 strain deposited with DSMZ on 13 January 2022; Accession No. DSM 34137, and *Thermoanaerobacterium sp.* BRSS1 strain deposited with DSMZ on 13 January 2022; Accession No. DSM 34145, all deposited in the name of Aix-Marseille University, 163 Avenue de Luminy 13288 Marseille Cedex 09, France. Preferably, said bacteria strains are cultured at a temperature higher than 55°C, preferably between 55 to 75°C.

In a preferred embodiment, said substrate comprises biomass selected from the group consisting of: sewage, agricultural waste products, brewery grain by-products, food waste, organic industrial waste, forestry waste, crops, grass, seaweed, plankton, algae, fish, fish waste, corn, potato waste, sugar cane waste, sugar beet waste, straw, paper waste, chicken manure, cow manure, hog manure, switchgrass and combinations thereof, more preferably fish waste or paper waste. In another preferred embodiment, said substrate may comprise starch and/or be supplemented with yeast extract or peptone such as tryptone.

In another aspect, the present disclosure relates to the use of a bacteria selected from the group consisting of: *Marinitoga sp.* BP5C14 (strain deposited with DSMZ on 10 February 2022; Accession No. DSM 34310), *Thermotoga sp.* GM6-5-3 strain (deposited with DSMZ on 13 January 2022; Accession No. DSM 34137), and *Thermoanaerobacterium sp.* BRSS1 (strain deposited with DSMZ on 13 January 2022; Accession No. DSM 34145) for producing hydrogen from substrates at pH lower than 6, preferably said substrate comprises biomass selected from the group consisting of sewage, agricultural waste products, straw, corn stover, brewery grain by-products, food waste, organic industrial waste, forestry waste, crops, grass, seaweed, plankton, algae, fish, fish waste, corn, potato waste, sugar cane waste, sugar beet waste, straw, paper waste, chicken manure, cow manure, hog manure, switchgrass and combinations thereof, more preferably fish waste or paper waste. In another preferred embodiment, said substrate may comprise starch and be supplemented with yeast extract or peptone such as tryptone.

The present disclosure also relates to a *Marinitoga sp.* BP5C14 strain (deposited with DSMZ on 10 February 2022; Accession No. DSM 34310), a *Thermotoga sp.* GM6-5-3 (strain deposited with DSMZ on 13 January 2022; Accession No. DSM 34137) or a *Thermoanaerobacterium sp.* BRSS1 strain deposited with DSMZ on 13 January 2022 Accession No. DSM 34145).

### DETAILED DESCRIPTION OF THE INVENTION

### New thermophilic bacteria strains

The present disclosure relates to thermophilic bacteria strains identified by the inventors that can be used to produce hydrogen under acidic conditions. The thermophilic bacteria of the present disclosure are selected from the group consisting of the *Marinitoga sp.* BP5C14 strain deposited on 10 February 2022 with the Deutsche Sammlung von Mikorganismen und Zellkulturen GmBH (DSMZ); Accession No. DSM 34310, *Thermotoga sp.* GM6-5-3 strain deposited with DSMZ on 13 January 2022; Accession No. DSM 34137), and *Thermoanaerobacterium sp.* BRSS1 strain deposited with DSMZ on 13 January 2022 Accession No. DSM 34145, all deposited in the name of Aix-Marseille University, 163 Avenue de Luminy 13288 Marseille Cedex 09, France.

Thermophilic bacteria are microorganisms capable to grow at temperatures higher than 50°C (See e.g., J.G. Black, Microbiology Principles and Applications, 2d edition, Prentice Hall, New Jersey, [1993] p. 145-146; Dworkin, M., Falkow, S., Rosenberg, E, Schleifer, K-H., Stackebarndt E. (eds) The prokaryotes, third edition, volume 3, p. 3-28296 and p. 797- 814 and p. 899- 924; Madigan M., Martinko, J. Brock Biology of Microorganisms, eleventh edition, p. 430 -441 and 414-415). The environments in which thermophiles may be isolated vary, although primarily associated with high temperatures and geothermal activity such as deep or shallow marine hydrothermal vents or continental hot springs. Such habitats have a worldwide distribution and are primarily associated with tectonically active zones and volcanic activity.

According to the present disclosure, said thermophilic bacteria belong to the Thermotogales order (phylum Thermotogae) or Thermoanaerobacterales order (phylum Bacillota).

Thermotogales are Gram-negative, anaerobic bacteria morphologically characterized by a typical sheath-like outer structure named "toga" surrounding the cells. The genus Thermotoga contains exclusively thermophilic species mostly isolated from marine hydrothermal environments. Thermoanaerobacterales contain spore-forming, Grampositive, saccharolytic, thermophilic, anaerobic bacteria.

According to the present disclosure, said thermophile bacteria strains are *Thermotoga sp.* GMS6 or *Marinitoga sp.* BP5CS14 or *Thermoanaerobacterium sp.* BRSS1 strain that have been screened for their ability to produce hydrogen under acidic condition (pH lower than 6.0).

In a particular aspect, the present disclosure relates to the use of GMS6, BRSS1 and BP5CS14 strains as described to produce hydrogen from a substrate, in particular at a pH lower than 6.

As used herein, the term "substrate" refers to any organic material, mixture, combination, derivative, or residual thereof that may be digested, preferably under anaerobic condition to produce hydrogen.

Substrates that have been used for producing hydrogen include carbohydrates, such as glucose, sucrose, xylose, cellulose, and starch, as well as organic wastes.

In a preferred embodiment, said substrate is biomass. Biomass is renewable organic material that comes from plants and animals. In a preferred embodiment, biomass is wood waste, crops and waste material, biogenic materials in municipal solid wastes, animal manures, and human sewage. In a more preferred embodiment, biomass is selected from the group consisting of sewage, agricultural waste products like corn steep liquor and soybean hulls, brewery grain by-products, food waste, organic industrial waste, forestry waste, crops, grass, seaweed, plankton, algae, fish, fish waste, dairy residues such as whey, cellulose and lignocellulosic containing materials such as wood, grass shrubs, perennial crops or annual crops, cellulose or lignocellulosic containing waste products such as scrap wood, reclaimed wood, sawdust, waste materials from potato, sugar cane, corn and/or sugar beet processing, residual material from biogas production, for example, corn and corn/manure mixtures, residues from silage, and combinations thereof.

The Inventors showed that the bacteria strains, according to the present disclosure are capable to produce hydrogen under acidic condition in the presence of fish waste such as salmon sludge or paper waste.

In a preferred embodiment said substrate is fish waste such as salmon sludge or paper waste, more preferably fish waste.

According to the invention, biomass may be derived from a single source, or biomass can comprise a mixture derived from more than one source.

In some embodiments, said substrate is supplemented with a nutritional supplement selected from the group consisting of a mineral source, vitamins, amino acids, and an energy source. In a preferred embodiment, the substrate is supplemented with yeast extract and/or peptone such as tryptone.

In another particular aspect, the present disclosure relates to a method for producing hydrogen using the thermophilic bacteria strains as described above in the presence of a substrate as described above, preferably fish or paper waste, more preferably salmon sludge.

The general aspects of culturing bacteria with substrate as well as the equipment and apparatus needed are known to the ordinarily skilled artisan or can be readily determined, whether on the laboratory or industrial scale. Such general aspects include preparation of the substrate (e.g., biomass), introduction of the substrate and any other media into a reactor or vessel, maintaining cultures and stocks of the strains, timing of inoculation, amounts of an inoculum, the form of the inoculum (e.g., from exponentially growing cultures or from lagphase cultures and otherwise), purification of the hydrogen from the culture mixture.

In a preferred embodiment, to produce hydrogen gas, substrate is cultured with GMS6, BRSS1, and BP5CS14 strains as described above under anaerobic conditions.

As used herein, the term "under anaerobic conditions" refers to reaction conditions for one or more reactions conducted in the absence or near absence (e.g., trace amounts) of oxygen, e.g., wherein a substrate (e.g., biomass) is introduced into a reactor vessel containing a bacterial strain and digested by "anaerobic digestion."

As used herein, "anaerobic digestion" refers to a series of processes in which bacteria break down a substrate in the absence of or near lack of oxygen to form hydrogen, among other byproducts.

According to the method of the present disclosure, the GMS6, BRSS1, and BP5CS 14 strains, as described above, are cultured with the substrate in temperature conditions allowing the bacteria to grow and produce hydrogen. One skilled in the art can determine a suitable and optimal growth temperature for conducting the methods of the invention. In a preferred embodiment, for culturing the bacteria strains as described above, the temperature is higher than 55°C, preferably between 55 to 75°C.

The pH level may be set at a predetermined pH at the beginning of the reaction without subsequent monitoring or maintenance during the process.

According to the present disclosure, the pH range to produce hydrogen according to the present disclosure is lower than pH 6, preferably may range from about pH 4.5 to about pH 6, preferably from about pH 5 to about pH 6.

The pH level may be controlled by maintaining natural buffering capacity, adding buffering chemicals, or by a pH controller, which automatically drive the pump used to inject alkaline or buffer solutions into the fermentation.

The time during which the substrate and the bacteria strains are in contact and produce hydrogen may be from about one to about 16 days, about one day to about 30 days, about one to about 20 days, or about two days to about 16 days.

Hydrogen may be collected using techniques well-known in the art.

In other embodiments, biogas may be collected in the headspace of the anaerobic digester under a floating or fixed biogas collection cover. For example, collection covers may be used as reservoirs for hydrogen storage at constant/low pressures. Further, volumetric or wet-tip gas meters may be used to measure gas production.

According to the present disclosure, the method may be performed in a bioreactor, preferably maintained under anaerobic conditions.

As used herein, the term "bioreactor" refers to an enclosed or isolated system for the containment of a bacteria strain as described above and a substrate. The "bioreactor" may preferably be configured for the anaerobic growth of the bacteria.

Said bioreactor may be configured to receive substrate and a bacteria strain as described above; a gas collector configured to collect hydrogen produced in the reactor vessel from the digestion of the substrate; optionally, a pH and temperature monitor/controller configured to monitor and control pH and temperature of the contents of the bioreactor.

Embodiments of the present disclosure are described in the following specific examples, which are exemplary only and not to be construed as limiting.

### EXAMPLES

### I. Cultures performed in flasks

### Marinitoga sp. BP5C14

In this project, the inventors are searching for a strain that grew well at a low pH and high temperature, so they checked all the strains of the collection potentially interesting on their respective optimal medium at different conditions of pH and temperatures: from pH 4 to pH 7 and from 55 °C to 80 °C.

The inventors first identified and selected a new strain, ***Marinitoga** sp.* BP5C14 that grew up to pH 5.5 at a temperature comprised between 55 and 65°C

### a) Batch 1: BP5C14 with starch

The inventors performed cultures using an optimal medium enriched with 0.5 or 1 g.L⁻¹ of yeast extract and the substrate to test (5% to 15%).

*Marinitoga sp.* BP5C14 was grown in an optimal medium at pH 5.5 with 0.3 % starch previously sterilized by autoclave. They added 1 g.L⁻¹ yeast extract and incubated the flasks with 10 % inoculum at 55°C for 15 days. The medium was stirred at 100 rpm.

**Table 1: Number of cells/mL during the 15 days incubation from the Marinitoga sp. BP5C14 culture with 0.3 % starch at pH 5.5 and 55°C in an autoclaved standard medium in a bioreactor with 1 g.L⁻¹ yeast extract, steered at 100 rpm.**

| **Time** | **T0** | **T1** | **T2** | **T3** | **T6** |
|---|---|---|---|---|---|
| **Number of cells/mL** | 1.²E+07 | 1.⁰E+07 | 2.⁰E+07 | 1.⁸E+07 | 4.⁴E+07 |
| **Comment** | NA | NA | NA | NA | NA |
| **Time** | **T9** | **T10** | **T13** | **T15** | |
| **Number of cells/mL** | 5.⁰E+07 | 3.⁰E+07 | 1.⁴E+08 | 1.³E+08 | |
| **Comment** | 0.5 g.L⁻¹ of yeast extract and 3 g.L⁻¹ of Tryptone added | NA | 100 mL of 0.3 % starch medium added | NA | |

The cells grew well from 1.2.10⁷ to 1.3.10⁸ cells/mL.

Tryptone addition induces an increase in hydrogen production. BP5C14 reached here 10 mL in the gas phase with acetic fermentation. There is some acetate produced after verification by HPLC.

**Table 2: Summary of the results of the accumulated H₂ and CO₂ produced (mM), the maximal production of H₂ and CO₂ (mmol/h/L), and the maximal percentage of H₂(%) produced during the 15-days incubation from the Marinitoga sp. BP5C14 culture with 0.3 % starch at pH 5.5 and 55°C in an autoclaved standard medium in a bioreactor with 1 g.L⁻¹ yeast extract, steered at 100 rpm.**

| | |
|---|---|
| **Cumulative H₂ production (mM)** | **51.74** |
| **Cumulative CO₂ production (mM)** | **32.36** |
| **Maximal production of H₂ (mmol/h/L)** | **0.73** |
| **Maximal production of CO₂ (mmol/h/L)** | **0.43** |
| **Maximal percentage of H₂ (%)** | **1.47** |

The maximal production of H₂ reaches 1.47 %. The cumulative H₂ production reaches 51.74 mM. BP5C14 can grow in a bioreactor on an optimal medium with starch. The ratio H₂/CO₂ is 1.60.

### b) Batch 2: BP5C14 with starch

The inventors cultivated *Marinitoga sp.* BP5C14 a second time in an autoclaved optimal medium at pH 5.5 with 0.3 % starch. They added 1 g.L⁻¹ yeast extract and incubated the flasks with 30 % inoculum from the first batch at 55°C for six days. The medium was stirred at 100 rpm.

**Table 3: Number of cells/mL during the one-week incubation from the second Marinitoga sp. BP5C14 culture with 0.3 % starch at pH 5.5 and 55°C in an autoclaved standard medium in a bioreactor with 1 g.L⁻¹ yeast extract, steered at 100 rpm.**

| **Time** | **T0** | **T1** | **T2** | **T5** | **T6** |
|---|---|---|---|---|---|
| **Number of cells/mL** | 5.⁰E+07 | 1.²E+08 | 6.⁹E+08 | 1.⁰E+08 | 5.⁰E+07 |
| **Comment** | NA | 2 g.L-1 of Tryptone added | 300mL of 1 % starch medium and 0.5 g.L⁻¹ of Tryptone added | 1 g.L-1 of | NA |
| | | | | Tryptone | |
| | | | | added | |

The second culture allows us to check the capacity of the strain BP5C14 to grow in a bioreactor. The strain grew up to 6.9.10⁸ cells/mL in an optimal medium with starch and Tryptone. Addition of Tryptone may promote strain growth and production of hydrogen.

**Table 4: Resume of the results of the accumulated produced H₂ and CO₂ (mM), the maximal production of H₂ and CO₂ (mmol/h/L) and the maximal percentage of H₂ produced (%) during the one-week incubation from the second Marinitoga sp. BP5C14 culture with 0.3 % starch at pH 5.5 and 55°C in an autoclaved standard medium in a bioreactor with 1 g.L⁻¹ yeast extract, steered at 100 rpm.**

| | |
|---|---|
| **Cumulative H₂ production (mM)** | **52.77** |
| **Cumulative CO₂ production (mM)** | **32.30** |
| **Maximal production of H₂ (mmol/h/L)** | **1.66** |
| **Maximal production of CO₂ (mmol/h/L)** | **1.00** |
| **Maximal percentage of H₂ (%)** | **2.93** |

The maximal production of H₂ reaches 2.93 %, more than the first time, surely because the inoculum originates from the first batch. There were more cells in the inoculum and these ones were used for the bioreactor. The ratio H₂/CO₂ is 1.63.

### c) Batch 3: BP5C14 with starch and Salmon sludge

The inventors cultivated a third time the *Marinitoga sp.* BP5C14 in an autoclaved optimal medium at pH 5.5 with 0.3 % starch. This time they added 2 g.L⁻¹ salmon sludge and 1 g.L⁻¹ yeast extract. They incubated the flasks with 30 % inoculum from the second batch at 55°C for six days. The medium was stirred at 100 rpm.

**Table 5: Number of cells/mL during the one-week incubation from the third Marinitoga sp. BP5C14 culture with 0.3 % starch, 0.2 % salmon sludge and 0.1 % yeast extract at pH 5.5 and 55°C in an autoclaved standard medium in a bioreactor steered at 100 rpm.**

| **Time** | **T0** | **T2** | **T3** | **T6** |
|---|---|---|---|---|
| **Number of cells/mL** | 2.0E+07 | 5.5E+08 | 2.3E+08 | 1.4E+08 |
| **Number of contaminant cells/mL** | 0.0E+00 | 0.0E+00 | 0.0E+00 | 7.2E+07 |
| **Percentage of contamination (%)** | 0.00 | 0.00 | 0.00 | 34.29 |
| **Comment** | 3 g.L⁻¹ of Tryptone added | 2 g.L⁻¹ of Tryptone added | 250mL of 0.5 % of starch medium, 0.2 % of salmon sludge, and 3 g.L⁻¹ of Tryptone added | NA |

The strain BP5C14 grew well with salmon sludge and starch. The cells increased from 2.10⁷ cells/mL to 7.2.10⁸ cells/mL. However, at the end of the batch, a contaminant appears.

The strain grew the same way with salmon sludge and starch as with only starch as a substrate.

**Table 6: Resume of the results of the accumulated produced H₂ and CO₂ (mM), the maximal production of H₂ and CO₂ (mmol/h/L), and the maximal percentage of H₂ and CO₂ produced (%) during the one-week incubation from the third Marinitoga sp. BP5C14 culture with 0.3 % starch, 0.2 % salmon sludge and 0.1 % yeast extract at pH 5.5 and 55°C in an autoclaved standard medium in a bioreactor steered at 100 rpm.**

| | |
|---|---|
| **Cumulative H₂ production (mM)** | **78.57** |
| **Cumulative CO₂ production (mM)** | **51.25** |
| **Maximal production of H₂ (mmol/h/L)** | **2.07** |
| **Maximal production of CO₂ (mmol/h/L)** | **0.78** |
| **Maximal percentage of H₂ (%)** | **7.51** |
| **Maximal percentage of CO₂ (%)** | **> 2** |

There was significantly more hydrogen production with salmon sludge. This culture reaches 7.51 % of H₂ produced. This pic seems to correspond to the apparition of the contaminant. The ratio H₂/CO₂ is 1.53.

### d) Batch 4: BP5C14 with starch and Salmon sludge

The inventors cultivated the *Marinitoga sp.* strain BP5C14 in an autoclaved optimal medium at pH 5.5 with 0.3 % Amidon. They added 2 g.L⁻¹ of salmon sludge and 1 g.L⁻¹ of yeast extract. They incubated the flasks with 20 % inoculum from the third batch at 55°C for one week. The medium was stirred at 100 rpm.

**Table 7: Number of cells/mL during the one-week incubation from the fourth Marinitoga sp. BP5C14 culture with 0.3 % starch, 0.2 % salmon sludge and 0.1 % yeast extract at pH 5.5 and 55°C in an autoclaved standard medium in a bioreactor steered at 100 rpm.**

| **Time** | **T0** | **T1** | **Tlbis** | **T2** |
|---|---|---|---|---|
| **Number of cells/mL** | 4.2E+07 | 4.2E+07 | 1.4E+07 | 1.4E+07 |
| **Number of contaminant cells/mL** | 2.2E+07 | 2.3E+08 | 4.2E+07 | 2.4E+07 |
| **Percentage of contamination (%)** | 34.38 | 84.78 | 75.00 | 63.16 |
| **Comment** | 4 g.L-1 of Tryptone added | Temperature increases up to 60°C | Temperature increases up to 70°C, 3 g.L-1 of Tryptone added | Temperature decreases to 60°C |

| **Time** | **T3** | **T4** | **T7** | |
|---|---|---|---|---|
| **Number of cells/mL** | 1.2E+07 | 2.0E+06 | 4.0E+06 | |
| **Number of contaminant cells/mL** | 1.2E+07 | 2.2E+07 | 3.8E+07 | |
| **Percentage of contamination (%)** | 50.00 | 91.67 | 90.48 | |
| **Comment** | 3 g.L-1 of Tryptone added | 200mL of 0.5 % starch medium, 0.5 % of salmon | NA | |
| | | sludge, 0.1 % of yeast extract and 0.3 % of | | |
| | | Tryptone replace | | |

The inventors stimulate pasteurization with a progressive increase of the temperature to 70°C to try to stop the growth of the contaminant from the salmon sludge. However, the contaminant overpowers *Marinitoga sp.* strain even after decreasing the temperature to 55°C again. In the end, 90.48% of the cells in the culture belong to the contaminant.

The contaminant survives at a temperature of 70°C on the contrary to the *Marinitoga sp.* strain. After decreasing the temperature, the contaminant grew again and produces up to 5mM/L/h of hydrogen when Tryptone is added.

**Table 8: Resume of the results of the accumulated produced H₂ and CO₂ (mM), the maximal production of H₂ and CO₂ (mmol/h/L), and the maximal percentage of H₂ produced (%) during the one-week incubation from the fourth Marinitoga sp. BP5C14 culture with 0.3 % starch, 0.2 % salmon sludge and 0.1 % yeast extract at pH 5.5 and 55°C in an autoclaved standard medium in a bioreactor, without Thiosulfate, steered at 100 rpm.**

| | |
|---|---|
| **Cumulative H₂ production (mM)** | **60.31** |
| **Cumulative CO₂ production (mM)** | **39.92** |
| **Maximal production of H₂ (mmol/h/L)** | **8.41** |
| **Maximal production of CO₂ (mmol/h/L)** | **2.65** |
| **Maximal percentage of H₂ (%)** | **25.83** |

The inventors reached the maximal percentage of hydrogen production (25.83 %). It is due to the contaminant which overpowers *Marinitoga sp.* strain. This strain is of interest as it produces more hydrogen, survives through a temperature of 70°C, grew at pH 5.5, and 60°C. The ratio H₂/CO₂ is 1.51.

The inventors isolated the contaminant on the same medium as the *Marinitoga sp.* They added 3 g.L⁻¹ Tryptone, 1 g.L⁻¹ yeast extract and incubated the culture at 60°C and pH 5.5. They extracted the DNA and sequenced the 16S to define the species' contaminant. It is close to 99.12 % to two *Thermoanaerobacterium thermosaccharolyticum* strains.

### II. Cultures performed in bioreactor

### 1. Experimental material and bioreactor used for growing experiments

All strains were batch cultured in a 2.3 L double-jacket glass bioreactor (FairMenTec, France) with a 1.5 L working volume. The Bioreactor was run with stirring driven by two axial impellers and was equipped with sensors to monitor temperature (Prosensor pt 100, France), pH (Mettler Toledo InPro 3253, Switzerland), and redox potential (Mettler Toledo InPro 3253, Switzerland). The incoming gas stream (O2-free N2 or O2-free N2 and H2), prepared via one or two mass-flow meters (Bronkhorst, range 0-100 or 0-100 and 0-10 mL min-1, Netherland, respectively), was injected through a nozzle immersed in the bioreactor. The steam in the outgoing gas stream was condensed in a water-cooler glass exhaust [temperature controlled at 4°C with a cooling bath equipped with a pump (Julabo SE 6, France)] to prevent liquid loss in the bioreactor (water-vapor condensates were returned to the culture vessel). On the outgoing gas streamline, downstream from the water-cooler glass exhaust, a micro-GC, equipped with a catharometric detector (MS5A, SRA Instrument, France), a GC-FPD, equipped with a flame photometric detector (PR 2100, Perichrom, France), and a CARBOCAP CO2 probe (Vaisala GMT 221, Finland), allowed online measurement of H2, N2 (micro-GC), H2S (GC-FPD), and CO2 (Probe) contents (see below for the analytic conditions). To prevent air from entering the bioreactor, the outgoing gas streamline was closed off by a hydraulic seal (2 cm deep immersion in oil). The bioreactor was heated by hot-water circulation in the double jacket using a heated bath equipped with a pump (Julabo F25, France). Bioreactor liquid volume and NaOH consumption, which was used to regulate culture pH, were followed via two scales [Sartorius Combics 1 and BP 4100 (France), respectively]. Temperature, pH, gas stream flow rates, and stirrer speed were regulated through control units (local loops). The bioreactor was connected to two pumps dedicated to the supply of fresh culture medium and to empty the reactor. All this equipment was connected to a Wago PLC (France) via a serial link (RS232/ RS485), a 4-20 mA analog loop or a digital signal. The PLC was connected to a computer for process monitoring and data acquisition. BatchPro software (Decobecq Automatismes, France) was used to monitor and manage the process with good flexibility and total traceability.

### 2. Results with the contaminant: Thernwanaerobacterium thermosaccharolyticum BRSS1

### a) Batch 1: BRSS1 with 5 % salmon sludge

The inventors cultivated the BRSS1 strain in an optimal medium at pH 5.5 with 5 % salmon sludge. They added 0.5 g.L⁻¹ yeast extract but no thiosulfate and incubated the flasks with 20 % of BP5C14 inoculum at 55°C for five days. They didn't sterilize and stir the medium at 150 rpm.

**Table 9: Number of cells/ml during the first BRSS1 culture with 5 % salmon sludge and 0.05 % yeast extract at pH 5.5 and 55°C in a standard medium in a bioreactor steered at 150 rpm.**

| **Time** | **T0** | **T1** | **T4** | **T5** |
|---|---|---|---|---|
| **Number of cells/mL** | NA | 0.0E+00 | 1.5E+08 | NA |

The cells grew well on salmon sludge up to 1.5.10⁸ cells/mL.

There is up to 5.13 % production of hydrogen. It represents up to 3.64 mmol/h/L of H₂ produced. The strain grew more with salmon sludge and starch than with only starch as a substrate.

**Table 10: Resume of the results of the accumulated produced H₂ and CO₂ (mM), the maximal production of H₂ and CO₂ (mmol/h/L), and the maximal percentage of H₂ and CO₂ produced (%) during the one-week incubation of the first BRSS1 culture with 5 % salmon sludge and 0.05 % yeast extract at pH 5.5 and 55°C in a standard medium in a bioreactor steered at 150 rpm.**

| | |
|---|---|
| **Cumulative H2 production (mM)** | **9.50** |
| **Cumulative CO2 production (mM)** | **20.55** |
| **Maximal production of H2 (mmol/h/L)** | **2.88** |
| **Maximal production of CO2 (mmol/h/L)** | **3.64** |
| **Maximal percentage of H2 (%)** | **5.13** |
| **Maximal percentage of CO2 (%)** | **5.17** |

The maximal production of H₂ reaches 5.13 %. The cumulative H2 production reaches 9.50 mM, the ratio H₂/CO₂ is 0.46. The strain produced less than with starch and salmon sludge. It reached up to 8 mmol/h/L and 60 mM with the two substrates in the medium.

### b) Batch 2: BRSS1 with 5 % Salmon sludge

The inventors cultivated the BRSS1 strain a second time in an optimal medium at pH 5.5 with 5 % salmon sludge. They added 0.5 g.L⁻¹ yeast extract but no thiosulfate and incubated with 15 % inoculum from the first batch at 55°C for eight days. The medium was stirred at 150 rpm.

**Table 11 Number of cells/ml during the second BRSS1 culture with 5 % salmon sludge and 0.05 % yeast extract at pH 5.5 and 55°C in a standard medium in a bioreactor steered at 150 rpm.**

| **Time** | **T0** | **T1** | **T2** | **T8** |
|---|---|---|---|---|
| **Number of BRSS1 cells / mL** | NA | 4.0E+07 | NA | 2.6E+07 |

There is up to 18.26 mmol/h/L of hydrogen produced. The strain grew well. It produces up to 12.12 mmol/h/L with sucrose, an optimal substrate (P Kongjan, S O-Thong, I Angelidaki, Engineering in Life Sciences 13 (2), 118-125).

**Table 12: Resume of the results of the accumulated produced H₂ and CO₂ (mM), the maximal production of H₂ and CO₂ (mmol/h/L) and the maximal percentage of H₂ and CO₂ produced (%) during the one-week incubation of the second BRSS1 culture with 5 % salmon sludge and 0.05 % yeast extract at pH 5.5 and 55°C in a standard medium in a bioreactor steered at 150 rpm.**

| | |
|---|---|
| **Cumulative H2 production (mM)** | **14.58** |
| **Cumulative CO2 production (mM)** | **36.71** |
| **Maximal production of H2 (mmol/h/L)** | **18.26** |
| **Maximal production of CO2 (mmol/h/L)** | **51.76** |
| **Maximal percentage of H2 (%)** | **2.72** |
| **Maximal percentage of CO2 (%)** | **4.30** |

Hydrogen production was better with salmon sludge this time. The cumulative H₂ production reaches 14.58 mM instead of 9.50 mM. However, the ratio H₂/CO₂ decreased from 0.46 to 0.40.

### c) Batch 3: BRSS1 with 5 % Salmon sludge

The inventors cultivated the BRSS1 strain a third time in an optimal medium at pH 5.5 with 5 % of salmon sludge. They added 0.5 g.L⁻¹ of yeast extract but no thiosulfate and incubated with 25 % of the inoculum from the second batch at 55°C for five days. The medium was stirred at 150 rpm.

**Table 13: Number of cells/ml during the third BRSS1 culture with 5 % salmon sludge and 0.05 % yeast extract at pH 5.5 and 55°C in a standard medium in a bioreactor steered at 150 rpm.**

| **Time** | **T0** | **T1** | **T2** | **T5** |
|---|---|---|---|---|
| **Number of cells / mL** | 6.0E+06 | 1.3E+08 | 1.1E+08 | 1.2E+08 |

The strain BRSS1 grew well. The cells increased from 6.10⁶ to 1.2.10⁸ cells/mL.

**Table 14: Resume of the results of the accumulated produced H₂ and CO₂ (mM), the maximal production of H₂ and CO₂ (mmol/h/L) and the maximal percentage of H₂ and CO₂ produced (%) during the one-week incubation of the third BRSS1 culture with 5 % salmon sludge and 0.05 % yeast extract at pH 5.5 and 55°C in a standard medium in a bioreactor steered at 150 rpm.**

| | |
|---|---|
| **Cumulative H₂ production (mM)** | **10.35** |
| **Cumulative CO₂ production (mM)** | **51.35** |
| **Maximal production of H₂ (mmol/h/L)** | **3.10** |
| **Maximal production of CO₂ (mmol/h/L)** | **4.01** |
| **Maximal percentage of H₂ (%)** | **5.30** |
| **Maximal percentage of CO₂ (%)** | **5.17** |

The inventors reached with this culture up to 10.35 mM the of hydrogen production. There is up to 3.10 mmol/h/L of hydrogen produced. The ratio H₂/CO₂ decreased from 0.40 to 0.20. It is less than from the second batch but more than the first. To conclude, the inventors will try new batches with more salmon sludge.

### d) Batch 4: BRSS1 with 10 % salmon sludge

The inventors cultivated the BRSS1 strain in an optimal medium at pH 5.5 with 10 % salmon sludge. They added 0.5 g.L⁻¹ yeast extract but no thiosulfate and incubated at 55°C for eight days with 15 % inoculum from the precedent batch (batch 3 with 5 % salmon sludge). They didn't sterilize and stir the medium at 150 rpm.

**Table 15: Number of cells/ml during the first BRSS1 culture with 10 % salmon sludge and 0.05 % yeast extract at pH 5.5 and 55°C in a standard medium in a bioreactor steered at 150 rpm.**

| **Time** | **T0** | **T1** | **T2** | **T8** |
|---|---|---|---|---|
| **Number of cells** / **mL** | 1.2E+07 | 7.4E+07 | 1.1E+08 | 7.2E+07 |

The strain grew a bit. The cells increased from 1.2.10⁷ to 7.2.10⁷ cells/mL.

**Table 16: Resume of the results of the accumulated produced H₂ and CO₂ (mM), the maximal production of H₂ and CO₂ (mmol/h/L), the maximal production of dissolved inorganic carbon (mM) and the maximal percentage of H₂ produced (%) during the one-week incubation of the fourth BRSS1 culture with 10 % salmon sludge and 0.05 % yeast extract at pH 5.5 and 55°C in a standard medium in a bioreactor steered at 150 rpm.**

| | |
|---|---|
| **Cumulative H₂ production (mM)** | **16.16** |
| **Cumulative CO₂ production (mM)** | **121.38** |
| **Maximal production of H₂ (mmol/h/L)** | **1.85** |
| **Maximal production of CO₂ (mmol/h/L)** | **3.30** |
| **Maximal percentage of H₂ (%)** | **2.82** |
| **Maximal percentage of CO₂ (%)** | **4.57** |

The maximal production of H₂ reaches 2.82 % and 1.85 mmol/h/L. The cumulative H₂ production reaches 16.16 mM. The ratio H₂/CO₂ decreases to 0.13.

### e) Batch 5: BRSS1 with 10 % Salmon sludge

The inventors cultivated the BRSS1 strain in an optimal medium at pH 5.5 with 10 % salmon sludge. They added 0.5 g.L⁻¹ yeast extract but no thiosulfate and incubated at 55°C for six days with 10 % inoculum from the precedent batch. They didn't sterilize and stir the medium at 150 rpm.

**Table 17: Number of cells/ml during the second BRSS1 culture with 10 % of salmon sludge and 0.05 % of yeast extract at pH 5.5 and 55°C in a standard medium in a bioreactor steered at 150 rpm.**

| **Time** | **T0** | **T1** | **T2** | **T3** | **T6** |
|---|---|---|---|---|---|
| **Number of BRSS1 cells / mL** | NA | 4.9E+07 | 9.4E+07 | 7.0E+07 | NA |

The strain grew less than the first culture at 10 % salmon sludge (precedent batch, batch 4). The number of cells increases from 4.9.10⁷ to 7.0.10⁷ cells/mL

**Table 18: Resume of the results of the accumulated produced H₂ and CO₂ (mM), the maximal production of H₂ and CO₂ (mmol/h/L), the maximal production of dissolved inorganic carbon (mM) and the maximal percentage of H₂ produced (%) during the one-week incubation of the second BRSS1 culture with 10 % salmon sludge and 0.05 % yeast extract at pH 5.5 and 55°C in a standard medium in a bioreactor steered at 150 rpm.**

| | |
|---|---|
| **Cumulative H₂ production (mM)** | **16.14** |
| **Cumulative CO₂ production (mM)** | **107.20** |
| **Maximal production of H₂ (mmol/h/L)** | **1.46** |
| **Maximal production of CO₂ (mmol/h/L)** | **2.94** |
| **Maximal percentage of H₂ (%)** | **2.06** |
| **Maximal percentage of CO₂ (%)** | **3.80** |

The maximal production of H₂ reaches 2.06 % and 1.46 mmol/h/L. It is less than the precedent batch also at 10 % of salmon sludge. However, the cumulative H2 production is roughly the same with 16.14 mM because the ratio H₂/CO₂ increases from 0.13 to 0.15.

### f) Batch 6: BRSS1 with 15 % Salmon sludge

The inventors cultivated the BRSS1 strain in an optimal medium at pH 5.5 with 15 % salmon sludge. They added 0.5 g.L⁻¹ of yeast extract but no thiosulfate and incubated at 55°C for four days with 15 % inoculum from the precedent batch. They didn't sterilize and stir the medium at 150 rpm.

**Table 19: Number of cells/ml during the BRSS1 culture with 15 % salmon sludge and 0.05 % yeast extract at pH 5.5 and 55°C in a standard medium in a bioreactor steered at 150 rpm.**

| **Time** | **T0** | **T1** | **T2** | **T3** | **T4** |
|---|---|---|---|---|---|
| **Number of BRSS1 cells / mL** | 1.4E+07 | 6.4E+07 | 1.5E+08 | 1.4E+08 | NA |

The strain grew more than with only 10 % salmon sludge. The number of cells increases from 1.4.10⁷ to 1.4.10⁸ cells/mL

**Table 20: Resume of the results of the accumulated produced H₂ and CO₂ (mM), the maximal production of H₂ and CO₂ (mmol/h/L), the maximal production of dissolved inorganic carbon (mM) and the maximal percentage of H₂ produced (%) during the four-days incubation of the BRSS1 culture with 15 % salmon sludge and 0.05 % yeast extract at pH 5.5 and 55°C in a standard medium in a bioreactor steered at 150 rpm.**

| | |
|---|---|
| **Cumulative H₂ production (mM)** | **34.88** |
| **Cumulative CO₂ production (mM)** | **174.99** |
| **Maximal production of H₂ (mmol/h/L)** | **15.48** |
| **Maximal production of CO₂ (mmol/h/L)** | **2.98** |
| **Maximal percentage of H₂ (%)** | **9.80** |
| **Maximal percentage of CO₂ (%)** | **4.66** |

The maximal production of H₂ reaches 9.80 % and 15.48 mmol/h/L. The cumulative H₂ production reaches 34.88 mM. The ratio H₂/CO₂ increases from 0.15 to 0.20. The strain grew better with 15 % than with 10 % or 5 % salmon sludge.

### g) BRSS1 batches: HPLC results

The inventors followed the products metabolized by the BRSS1 strain from salmon sludge by HPLC.

In the beginning, the BRSS1 strain consumes glucose and fructose to produce lactate. Then, it produces butyrate, ethanol, and some propionate by using lactate. It also produces a bit of acetate. The metabolism was mostly an amino acid fermentation.

### 3. Results with Thermotoga sp. GM6 with comparison with T. maritima strains

The production rate of *Thermotoga maritima* is reported in existing articles, in particular:
- Boileau, C., Auria, R., Davidson, S. et al. Hydrogen production by the hyperthermophilic bacterium Thermotoga maritima part I: effects of sulfured nutriments, with thiosulfate as model, on hydrogen production and growth. Biotechnol Biofuels 9, 269 (2016)
- Auria, R., Boileau, C., Davidson, S. et al. Hydrogen production by the hyperthermophilic bacterium Thermotoga maritima Part II: modeling and experimental approaches for hydrogen production. Biotechnol Biofuels 9, 268 (2016).

The most used strain is the type strain MSB8T (DSMZ 3109). It is used regularly to produce hydrogen as it grows quickly, and the production rate is high. Generally, the standard culture medium contains glucose at pH 6.5.

The H₂ molar yields reported in the literature for *T. maritima* are included between 1.7 and 4.0 mol.mol⁻¹. In the bioreactor of the MIO platform, the yields on glucose at pH 6.5 are 2.0 and 2.2 mol mol⁻¹. In these conditions, the maximal production of H₂ for *T. maritima* is 5.6 mmol/L/h. It can reach up to 14 mmol/L/h. The maximal H₂ productivity of *Thermotoga maritima* is 7 mmol/L/h.

In this project, the inventors are searching for a strain that grew well at a low pH and high temperature and can produce hydrogen at a high rate.

The inventors grew different strains in Hungate tubes with 5 mL of their optimal medium. They add 0.8 g.L⁻¹ yeast extract, 10 mM Thiosulfate (except for the ***Marinitoga** sp.)* and 1 g.L⁻¹ Tryptone. After several subcultures, they checked the accumulated hydrogen (H₂) production by gas chromatography after two weeks.

**Table 21: Accumulated production of H₂ (mM) after 2 weeks (a) by the 7 Thermotoga sp. at pH 7 in their respective standard medium (0.8 g.L⁻¹ of yeast extract, 10 mM of Thiosulfate, 1 g.L⁻¹ of Tryptone) and 2 temperatures (65 °C and 80 °C for the Thermotoga sp).**

| **Temperature (°C)** | **65** | **80** |
|---|---|---|
| *T. maritima* | 0.11 mM | 0.30 mM |
| *T. neopolitana* | 0.20 mM | 0.38 mM |
| *T. hypogea* | 0.03 mM | 0.02 mM |
| *T. elfii* | 0.34 mM | 0.01 mM |
| *T. alcaliphila* | 0.51 mM | 0.03 mM |
| *GM6* | 0.26 mM | 0.02 mM |
| *M5M4* | 0.54 mM | 0.001 mM |

The strains were grown in Hungate tubes with 5 mL of their respective medium and their optimal conditions. 0.8 g.L⁻¹ yeast extract or not, 10 mM Thiosulfate and 0.3 % to 0.5 % of defined substrates were added. The inventors incubated the cultures in triplicates at 2 temperatures: 65 °C or 80 °C for the *Thermotoga sp* at pH 5. They check the growth two weeks after the inoculation.

**Table 22: Growth of the Thermotoga sp. grew on four different defined substrates (hemicellulose, cellulose, starch, xylan) in a standard medium with 10 mM Thiosulfate and with or without 0.8 g.L⁻¹ yeast extract at 2 temperatures (65 °C and 80 °C) and pH 5.**

| **Temperature (°C)** | **65** | | | | | | | |
|---|---|---|---|---|---|---|---|---|
| **Yeast extract (g.L⁻¹)** | **0** | | | | **0.8** | | | |
| | Hemicellulose | Cellulose | Starch | Xylan | Hemicellulose | Cellulose | Starch | Xylan |
| *T. maritima* | NO | NO | YES | YES | NO | NO | YES | YES |
| *T. alcaliphila* | NO | YES | NO | NO | NO | NO | NO | NO |
| *T. elfii* | NO | NO | NO | NO | NO | NO | NO | YES |
| *T. hypogea* | NO | NO | NO | NO | NO | NO | NO | NO |
| *T. neopolitana* | NO | NO | YES | YES | NO | NO | YES | YES |
| *GM6* | YES | NO | NO | NO | NO | NO | YES | NO |
| *M5M4* | NO | NO | NO | NO | NO | NO | NO | NO |

| **Temperature (°C)** | **80** | | | | | | | |
|---|---|---|---|---|---|---|---|---|
| **Yeast extract (g.L⁻¹)** | **0** | | | | **0.8** | | | |
| | Hemicellulose | Cellulose | Starch | Xylan | Hemicellulose | Cellulose | Starch | Xylan |
| *T. maritima* | NO | NO | YES | NO | NO | NO | YES | NO |
| *T. alcaliphila* | NO | NO | NO | NO | NO | NO | NO | NO |
| *T. elfii* | NO | NO | NO | NO | NO | NO | NO | NO |
| *T. hypogea* | NO | NO | NO | NO | NO | NO | NO | NO |
| *T. neopolitana* | NO | NO | YES | NO | NO | NO | YES | YES |
| *GM6* | NO | NO | NO | NO | NO | NO | NO | NO |
| *M5M4* | NO | NO | NO | NO | NO | NO | NO | NO |

*T. maritima, T. neapolitana, T. alcaliphila* and GM6 grew at pH 5. GM6 grew significantly the most.

The inventors tested and selected the best strains and substrates for hydrogen production on complex substrates. They tested six complex substrates with 0.5 % of the substrate in the optimal medium and pH for each strain: Salmon sludge, Straw, Cow manure, Maize silage, Steam exploded hard wood, Protamylasse.

The inventors add 10 mM Thiosulfate in the cultures. The cultures were incubated in triplicates in Hungate tubes with 5 mL of medium and different conditions:
- with or without 1 g.L⁻¹ yeast extract
- with or without autoclave
- at 2 temperatures: 65 °C and 80 °C

The inventors check the hydrogen produced by gas chromatography after two weeks. They look at the strain by microscopy to verify the contamination rate. Indeed, the control cultures without inoculum produce hydrogen.

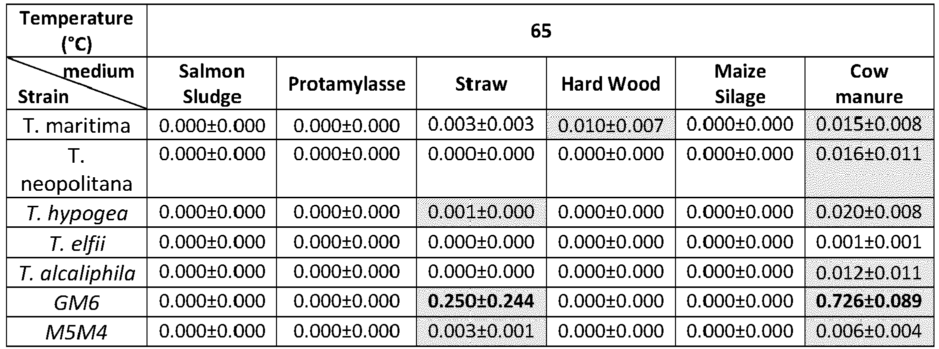

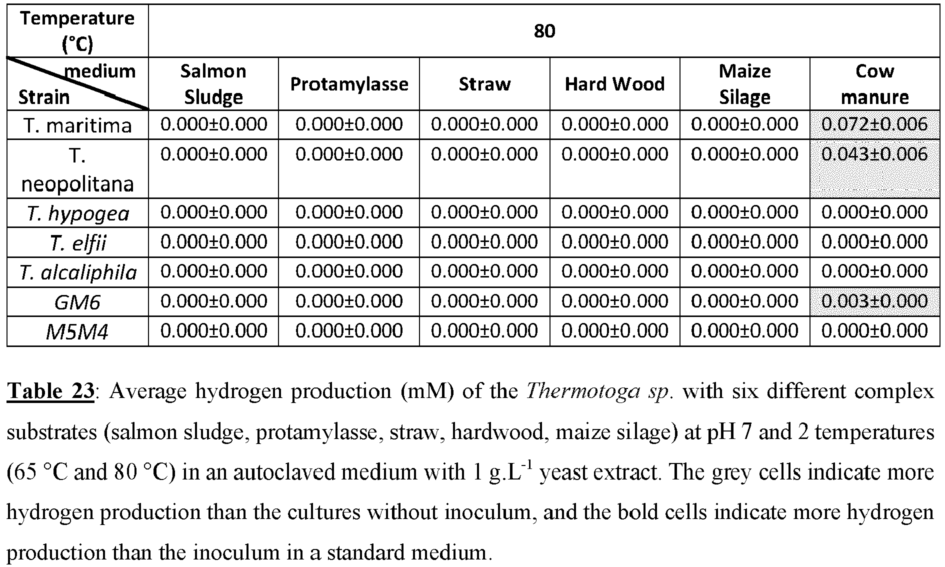

GM6 produce a higher quantity of hydrogen in comparison to other strains with cow manure and straw at 65°C. The control cultures didn't produce any hydrogen on maize silage and cow manure.

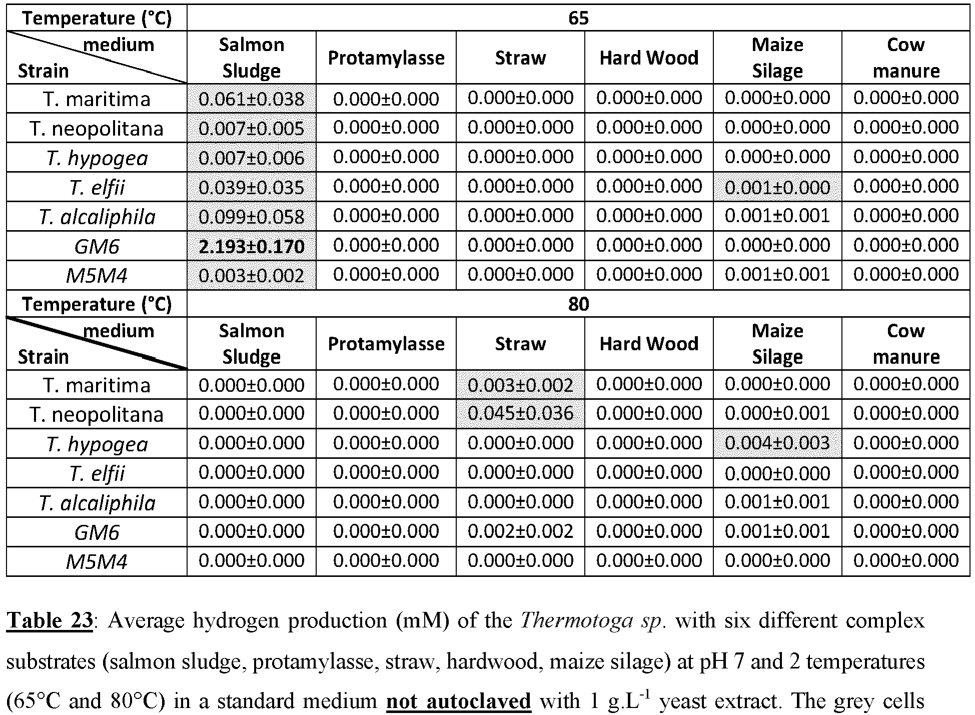

indicate more hydrogen production than the cultures without inoculum, and the bold cells indicate more hydrogen production than the inoculum in a standard medium.

Only GM6 produces hydrogen (up to 2.193 ± 0.170 mM) in a medium with salmon sludge. This strain is therefore of interest for the production of hydrogen.

As the inventors are looking for a strain capable of producing hydrogen at low pH; the production of hydrogen at a low pH with GM6 strain was then evaluated.

### a) Batch 1: GM6 with starch and Salmon sludge

The inventors cultivated the *Thermotoga sp.* GM6 in an autoclaved optimal medium at pH 5.5 with 0.3 % starch and 0.15 % Tryptone. They added 0.05 g.L⁻¹ yeast extract but no thiosulfate and incubated at 65°C for three days with 13 % inoculum. The medium was stirred at 150 rpm.

**Table 24: Number of cells/ml and comments during the three-days GM6 culture with 0.3 % starch, 0.15 % Tryptone and 0.05 % yeast extract at pH 5.5 and 65°C in a standard medium in a bioreactor steered at 150 rpm.**

| **Time** | **T0** | **T1** | **T2** | **T3** |
|---|---|---|---|---|
| **Number of GM6 cells / mL** | 2.0E+06 | NA | 3.5E+08 | NA |
| **Comment** | NA | 50mL of inoculum added (~ 10⁹) | NA | NA |

The cells grew well from 2.0.10⁶ to 3.5.10⁸ cells/mL.

**Table 25: Resume of the results of the accumulated H₂ and CO₂ produced (mM), the maximal production of H₂ and CO₂ (mmol/h/L) and the maximal percentage of H₂ and CO₂ produced (%) during the three-days incubation from the first Thermotoga sp. GM6 culture with 0.3 % starch, 0.15 % Tryptone and 0.05 % yeast extract at pH 5.5 and 65°C in an autoclaved standard medium in a bioreactor steered at 150 rpm.**

| | |
|---|---|
| **Cumulative H₂ production (mM)** | **50.58** |
| **Cumulative CO₂ production (mM)** | **31.66** |
| **Maximal production of H₂ (mmol/h/L)** | **2.29** |
| **Maximal production of CO₂ (mmol/h/L)** | **1.75** |
| **Maximal percentage of H₂ (%)** | **3.55** |
| **Maximal percentage of CO₂ (%)** | **2.51** |

The maximal production of H₂ reaches 3.55 % and 2.29 mmol/h/L. The cumulative H₂ production reaches 50.58 mM and the ratio H₂/CO₂ is 1.60. The strain can grow well in a bioreactor.

### b) Batch 2: GM6 with starch and Salmon sludge

The inventors cultivated a second time the *Thermotoga sp.* GM6 in an autoclaved optimal medium at pH 5.5 with 0.5 % starch. This time, they added 5 g.L⁻¹ salmon sludge and 0.05 g.L⁻¹ yeast extract but no thiosulfate. They incubated at 65°C for three days with 15 % inoculum from the first batch. The medium was stirred at 100 rpm.

**Table 26: Number of cells/ml during the three-days GM6 culture with 0.3 % starch, 0.5 % salmon sludge and 0.05 % yeast extract at pH 5.5 and 65°C in a standard medium in a bioreactor steered at 150 rpm.**

| **Time** | **T0** | **T3** |
|---|---|---|
| **Number of GM6 cells / mL** | NA | 8.4E+07 |

The strain grew less than with just starch in the medium.

**Table 27: Resume of the results of the accumulated H₂ and CO₂ produced (mM), the maximal production of H₂ and CO₂ (mmol/h/L) and the maximal percentage of H₂ and CO₂ produced (%) during the three-days GM6 culture with 0.3 % starch, 0.5 % salmon sludge and 0.05 % yeast extract at pH 5.5 and 65°C in a standard medium in a bioreactor steered at 150 rpm.**

| | |
|---|---|
| **Cumulative H₂ production (mM)** | **6.23** |
| **Cumulative CO₂ production (mM)** | **3.56** |
| **Maximal production of H₂ (mmol/h/L)** | **0.70** |
| **Maximal production of CO₂ (mmol/h/L)** | **0.45** |
| **Maximal percentage of H₂ (%)** | **1.89** |
| **Maximal percentage of CO₂ (%)** | **1.09** |

The maximal production of H₂ reaches 1.89 % instead of 3.55 % and 0.70 mmol/h/L instead of 2.29 mmol/h/L. The cumulative H₂ production reaches only 6.23 mM. Despite the ratio H₂/CO₂ that increases from 1.60 to 1.75, hydrogen production decreases greatly with salmon sludge in the medium.

### c) Results of hydrogen production on paper mills

The inventors incubated the two strains of interest (GM6 and BRSS1) in 50 mL flask containing an optimal medium at pH 5.5 with 5 % paper mills and 0.05 % yeast extract. They incubated two replicates and two controls at 55°C for BRSS1 and 65°C for GM6 and steered at 150 rpm.

The GM6 strain grew well with this substrate. It reaches up to 3.42 mM of hydrogen produced and 1.05.10⁸ cells/mL.

## Claims

1. A method for producing hydrogen comprising the step of culturing at least one thermophilic bacteria in the presence of a substrate at a pH lower than 6 and in temperature conditions allowing the bacteria to grow and produce hydrogen, wherein said thermophilic bacteria is selected from the group consisting of: *Marinitoga sp.* BP5C14 strain (deposited with DSMZ on 10 February 2022; Accession No. DSM 34310), *Thermotoga sp.* GM6-5-3 strain (deposited with DSMZ on 13 January, 2022; Accession No. DSM 34137), and *Thermoanaerobacterium sp.* BRSS1 strain (deposited with DSMZ on 13 January 2022; Accession No. DSM 34145).

2. The method of claim 1 wherein said bacteria strain is cultured at a temperature higher than 55°C, preferably between 55 to 75°C.

3. The method according to any one of claims 1 or 2 wherein said substrate comprises biomass selected from the group consisting of: sewage, agricultural waste products, brewery grain by-products, food waste, organic industrial waste, forestry waste, crops, grass, seaweed, plankton, algae, fish, fish waste, corn, potato waste, sugar cane waste, sugar beet waste, straw, paper waste, chicken manure, cow manure, hog manure, switchgrass and combinations thereof.

4. The method of claim 3 wherein said substrate comprises fish waste or paper waste.

5. The method according to any one of claims 1 to 4 wherein said substrate comprises starch.

6. The method according to any one of claims 1 to 5 wherein said substrate is supplemented with yeast extract or peptone such as tryptone.

7. The use of a bacteria selected from the group consisting of: *Marinitoga sp.* BP5C14 strain (deposited with DSMZ on 10 February 2022; Accession No. DSM 34310), *Thermotoga sp.* GM6-5-3 strain (deposited with DSMZ on 13 January, 2022; Accession No. DSM 34137), and *Thermoanaerobacterium sp.* BRSS1 strain (deposited with DSMZ on 13 January 2022; Accession No. DSM 34145) for producing hydrogen from substrate at pH lower than 6.

8. The use of claim 7 wherein said substrate comprises biomass selected from the group consisting of: sewage, agricultural waste products, straw, corn stover, brewery grain by-products, food waste, organic industrial waste, forestry waste, crops, grass, seaweed, plankton, algae, fish, fish waste, corn, potato waste, sugar cane waste, sugar beet waste, straw, paper waste, chicken manure, cow manure, hog manure, switchgrass and combinations thereof.

9. The use of claim 7 wherein said substrate comprises fish waste or paper waste.

10. The use according to any one of claims 7 to 9 wherein said substrate comprises starch.

11. The use according to any one of claims 7 to 10 wherein said substrate is supplemented with a yeast extract and/or peptone such as tryptone.

12. A *Marinitoga sp.* BP5C14 strain (deposited with DSMZ on 10 February 2022; Accession No. DSM 34310).

13. A *Thermotoga sp.* GM6-5-3 strain (strain deposited with DSMZ on 13 January 2022; Accession No. DSM 34137).

14. A *Thermoanaerobacterium sp.* BRSS1 strain deposited with DSMZ on 13 January 2022; Accession No. DSM 34145).
